(19) Europäisches Patentamt
European Patent Office
Office européen des brevets

(11) Publication number: **0 008 759**
**B1**

(12) # EUROPEAN PATENT SPECIFICATION

(45) Date of publication of patent specification: **01.12.82**

(51) Int. Cl.³: **C 07 D 231/56**
**//C07C101/10**

(21) Application number: **79103147.9**

(22) Date of filing: **27.08.79**

(54) **New process for preparing an 1H-indazol-3-ylacetic acid derivative.**

(30) Priority: **28.08.78 JP 105042/78**
**12.09.78 JP 112553/78**

(43) Date of publication of application:
**19.03.80 Bulletin 80/6**

(45) Publication of the grant of the patent:
**01.12.82 Bulletin 82/48**

(84) Designated Contracting States:
**AT BE CH DE FR GB IT LU NL SE**

(56) References cited:
**GB - A - 1 517 148**

**HETEROCYCLIC COMPOUNDS, vol. 22, 1967, pages 289—305 New York, U.S.A.**
**A. WEISSBERGER: "Pyrazoles, Pyrazolines, Pyrazolidines, Indazoles, and Condensed Rings"**

**CHEMICAL ABSTRACTS, vol. 83, no. 12, 22nd Septembter 1975, no. 10069g**
**Columbus, Ohio, U.S.A.**

**CHEMICAL ABSTRACTS, vol. 87, no. 7, 15th August 1977, page 473, no. 53276x**
**Columbus, Ohio, U.S.A.**

(73) Proprietor: **Fujisawa Pharmaceutical Co. Ltd.**
**3, Doshomachi 4-chome Higashi-ku**
**Osaka-shi, Osaka 541 (JP)**

(72) Inventor: **Kariyone, Kazuo**
**No. 8 Tojiinkita-machi**
**Kita-ku, Kyoto, (603) (JP)**
Inventor: **Yagi, Hideo**
**No. 3-27-23 Shinsenriminami-machi**
**Toyonaka, (565) (JP)**
Inventor: **Matsushima, Hiroshi**
**No. 2-9-15-302 Nishishoji**
**Minoh-shi, Osaka-prefecture (JP)**

(74) Representative: **Türk, Dietmar, Dr. rer. nat. et al,**
**Redies, Redies, Türk & Gille Patentanwälte**
**Brucknerstrasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 008 759 B1

New process for preparing an 1H-indazol-3-ylacetic acid derivative

This invention relates to new processes for preparing an 1H-indazol-3-ylacetic acid derivative of the formula:

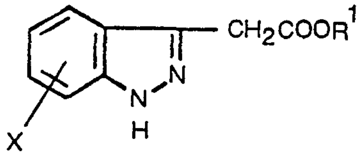

(I)

wherein $R^1$ is a hydrogen atom or a lower alkyl group, and X is a hydrogen atom, a halogen atom or a lower alkoxy group, or its salt.

An 1H-indazol-3-ylacetic acid derivative (I) or its salt excepting the compound wherein X is a lower alkoxy group, is a known compound as a plant growth regulator and especially a sodium salt of the compound wherein X is 5-chloro and $R^1$ is hydrogen, is marketed as "RUTIACE". And, various processes for preparation thereof are also known.

In the various known processes for preparation of the 1H-indazol-3-ylacetic acid derivative (I) or its salt, there is exemplified a method described in Japanese Patent Publication No. 82158/1975, Laid Open No. 5766/1977 as the most relevant prior art.

In said prior literature, the following methods are disclosed:

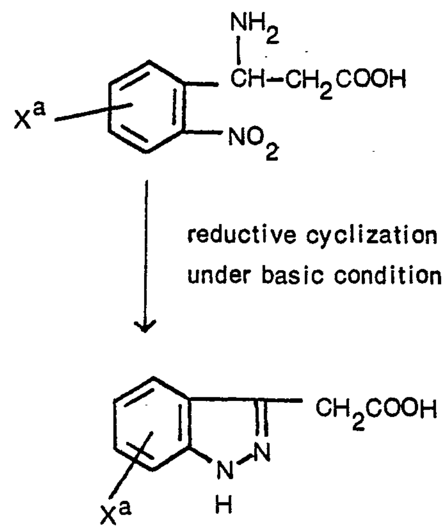


$(II^a)$

$(I^a)$

wherein $X^a$ is a hydrogen atom or a halogen atom.

(a) A process for preparing the 1H-indazol-3-ylacetic acid derivative $(I^a)$ which comprises reacting the aminoacetic acid derivative $(II^a)$ with a metal such as aluminum, zinc or the like under a basic condition.

(b) A process for preparing the 1H-indazol-3-ylacetic acid derivative $(I^a)$ which comprises reacting the aminoacetic acid derivative $(II^a)$ with an amalgam such as zinc amalgam, sodium amalgam or the like under a basic condition.

(c) A process for preparing the 1H-indazol-3-ylacetic acid derivative $(I^a)$ which comprises subjecting the aminoacetic acid derivative $(II^a)$ to a catalytic reduction in the presence of a metal catalyst such as palladium charcoal, platinum oxide, colloidal platinum or the like under basic condition.

However, the prior methods as above have not led to satisfactory results in an industrial manufacture of 1H-indazol-3-ylacetic acid derivative $(I^a)$ or its salt for the following reasons:

That is,

(1) In the methods (a) and (b), many complicated steps are often required for isolation of the 1H-indazol-3-ylacetic acid derivative $(I^a)$ or its salt from the reaction mixture including various metal salts. In addition it is desirable to recover such metal salts and amalgam rather than to discard them in view of the desirability of preventing pollution of the environment, and furthermore use of such an amalgam has possibly an adverse influence on working environment.

(2) The method (c) is necessarily attended with a by-product, e.g. carbostyryl derivative (6-chloro-1,2-dihydroquinolin-2-one) in the reaction mixture so that it is difficult to obtain the 1H-indazol-3-ylacetic acid derivative $(I^a)$ or its salt in high yield.

As results of extensive study into ways of overcoming the above mentioned problems, the present

inventors have now found a new process for preparation of 1H-indazol-3-ylacetic acid derivative (I) or its salt, by which the object compound (I) or its salt can be obtained in high yield without the above mentioned various problems.

Accordingly, this invention provides new processes for preparation of 1H-indazol-3-ylacetic acid derivative (I) or its salt, by which the object compound (I) or its salt can be obtained in higher yield and in higher purity than by prior processes so that the processes according to this invention are industrially advantageous.

According to this invention, 1H-indazol-3-ylacetic acid derivative (I) or its salt are prepared by the following method:

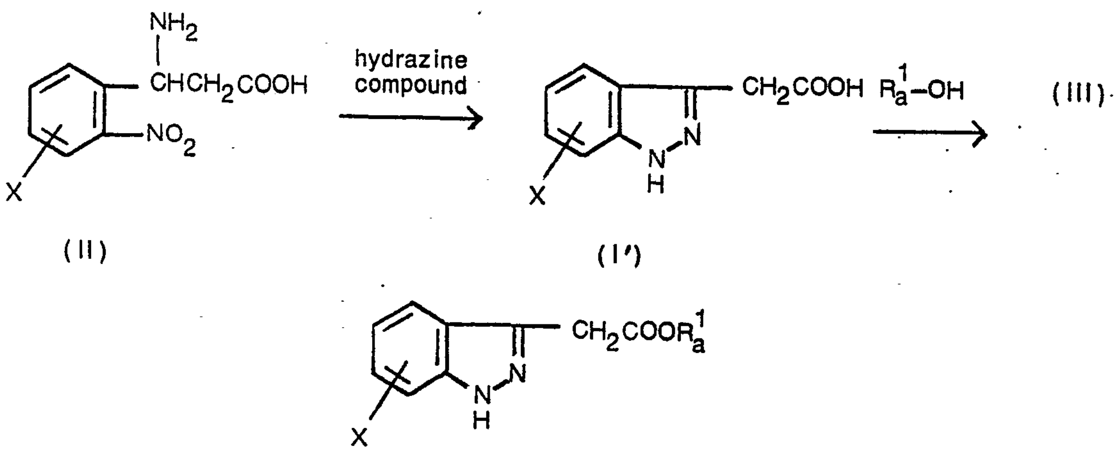


(II) (I') (I'')

wherein X is a hydrogen atom, a halogen atom, a lower alkoxy group, $R^1$ is a hydrogen atom or a lower alkyl group, $R_a^1$ is a lower alkyl group.

In this specification and claim, the term "lower" is intended to mean a group having 1 to 6 carbon atoms, unless otherwise indicated.

A lower alkyl group for $R^1$ is thus a straight or branched C1 to C6, preferably C1 to C4 and more preferably C1 to C2 alkyl group such as methyl, ethyl, propyl, isopropyl, butyl, isobutyl, *tert*-butyl and the like.

The halogen atom for X includes fluorine, chlorine, bromine and iodine and preferably chlorine and bromine.

A suitable example of alkoxy group for X includes C1 to C4 alkoxy such as methoxy, ethoxy, propoxy, butoxy and the like.

A suitable example of a salt of 1H-indazol-3-ylacetic acid derivative (I) includes a salt with an inorganic base, for example, a metal salt such as an alkali metal salt (e.g. sodium salt, potassium salt etc.) and alkaline earth metal salt (e.g. calcium salt, magnesium salt, etc.) or an ammonium salt.

Detailed explanation of processes for preparation of 1H-indazol-3-ylacetic acid derivative (I) or its salt will be made in the following:

(1): Compound (II) → Compound (I')

This process is conducted by reacting an aminoacetic acid derivative (II) or its salt with a hydrazine compound.

A hydrazine compound used in this reaction includes hydrazine (preferably hydrazine hydrate), arylhydrazine (e.g. phenylhydrazine etc.), alkylhydrazine (e.g. methylhydrazine etc.), hydrazide (e.g. acethydrazide etc.) and the like, or a salt thereof (e.g. hydrochloride, sulfate, etc.).

The reaction is usually carried out in an organic solvent such as water, alcohol (e.g. methanol, ethanol, propanol, isopropyl alcohol etc.), tetrahydrofuran, dioxane, or other conventional solvent which does not adversely influence to the reaction; or an optional mixture thereof.

The reaction is preferably carried out in the presence of a base such as alkali metal hydroxide (e.g. sodium hydroxide, potassium hydroxide etc.) or ammonia and the like. In such a case, a hydrazine compound itself to be used in this process is basic, and therefore it is not always necessary to add said base to the reaction mixture.

The reaction is preferably carried out in the presence of a catalyst such as activated charcoal, Raney nickel, palladium charcoal, platinum oxide, colloidal platinum or the like. Among such catalysts, activated charcoal is most suitable in this reaction.

The reaction is ordinarily carried out at ambient temperature, under warming or cooling.

Thus obtained 1H-indazol-3-ylacetic acid derivative (I') or its salt is isolated from the reaction mixture by a conventional manner.

(2): Compound (I') → Compound (I'')

This process includes a so-called "if-desired" process, which is conducted by reacting the 1H-indazol-3-ylacetic acid derivative (I') or its salt with a lower alkanol of the formula: $R_a^1OH$ (III) wherein $R_a^1$ is as defined above.

This esterification process is a process known per se and it is known in the case of the compound wherein X is a hydrogen atom or a halogen atom (e.g. Japanese publication no. 37270/1973).

This process is conducted by reacting a compound (I') or its salt with a compound (III).

The compound (III) can be used as a solvent in this reaction.

This reaction is preferably carried out in the presence of an inorganic acid such as hydrochloric acid, sulfuric acid etc.

(3): Preparation of the starting compound (II)

The starting compound (II) or its salt includes known compounds, i.e. ones wherein X is a hydrogen atom or a halogen atom (c.f. Japanese Patent Application No. 82158/1975, Laid Open No. 5766/1977) and new compounds, i.e. ones wherein X is an alkoxy group, which can be prepared according to substantially the same method as described in the above Japanese Patent Application.

Alternatively, the aminoacetic derivative (II) or its salt may be prepared by the method as shown the following schemes.

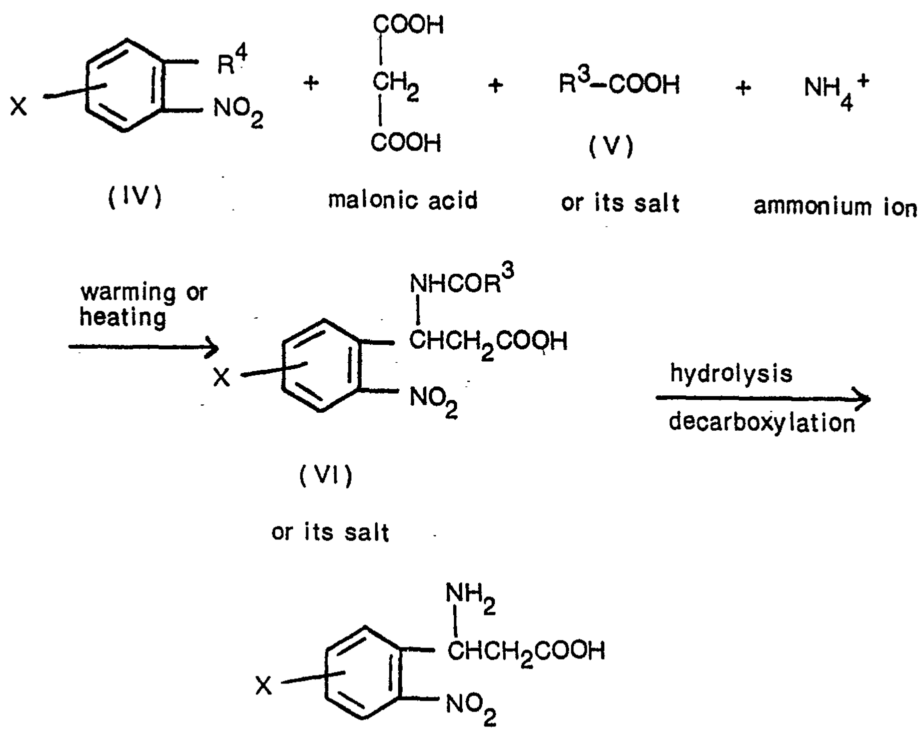


(IV) + malonic acid + (V) or its salt + ammonium ion

(VI) or its salt

(II) or its salt

wherein X is as defined above, $R^4$ is a diacetoxymethyl group and $R^3$ is a hydrogen atom or a lower alkyl group.

In above definitions, suitable examples of lower alkanoic acid ($R^3$—COOH) (V) is exemplified as formic acid, propionic acid, butylic acid, pivalic acid etc.

Ammonium ion may be added in the form of ammonium salt of said lower alkanoic acid (V) or ammonia gas.

The process from the compound (IV) to the compound (VI) is preferably conducted under warming or heating, if desired, with an addition of suitable solvent(s).

The thus obtained compound (VI) is subjected to hydrolysis usually without isolation thereof. Hydrolysis is conducted by conventional method, preferably, by an addition of an acid such as hydrochloric acid, sulfuric acid etc. and preferably refluxed in hydrochloric acid.

A suitable example of a salt of the aminoacetic acid derivative (II) includes the same one mentioned above as a salt of 1H-indazol-3-ylacetic acid derivative (I).

In addition, acid addition salt is also included, suitable example of which is an inorganic acid salt (e.g. hydrochloride, hydrobromide, sulfate, phosphate, carbonate, bicarbonate etc.), an organic carboxylic or sulfonic acid salt (e.g. acetate, maleate, lactate, tartrate, mesylate, benzenesulfonate, tosylate, etc.), an amino acid salt (e.g. arginine salt, aspartic acid salt, glutamic acid salt, lysine salt, serine salt, etc.) and the like.

The following examples are given for illustrating the present invention in more detail.

## Example 1

*Preparation of 5-chloro-1H-indazol-3-ylacetic acid*

Hydrazine (mono hydrate) (2.5 g) was added to a solution of 3-amino-3-(5-chloro-2-nitrophenyl)propionic acid (15 g) in 5% aqueous sodium hydroxide solution (75 ml). Raney nickel catalyst (about 10 mg) was added to the solution at 80°C with vigorous stirring and the mixture was stirred at 80—85°C for 10 minutes. After additional Raney nickel (10 to 20 mg) was added to the mixture, the stirring was continued at the same temperature until evolution of nitrogen gas ceased (it took about 20 minutes). The reaction mixture was filtered. The filtrate was acidified to pH 2 with hydrochloric acid, and cooled to afford crystals, which were filtered and washed with water. The crystals were dissolved in 5% warm aqueous sodium bicarbonate solution (75 ml) and the solution was filtered, acidified to pH 2 with hydrochloric acid and cooled to afford a pale yellow crystals. The pale yellow crystals were collected by filtration, washed with water and dried to give 5-chloro-1H-indazol-3-ylacetic acid (12 g, yield 93%). M.p. 209—210°C (dec.)

## Example 2

*Preparation of 6-chloro-1H-indazol-3-ylacetic acid*

(1) Preparation of 3-amino-3-(4-chloro-2-nitrophenyl)propionic acid

$\alpha,\alpha$-Diacetoxy-4-chloro-2-nitrotoluene (10 g), malonic acid (4.8 g) and ammonium acetate (6.8 g) were dissolved in a mixture of formic acid (5.2 ml) and acetic acid (10 ml). The solution was stirred at 80—85°C for 2 hours, 90—95°C for 2 hours, 95—100°C for 1 hour and then heated under reflux for 1 hour. Then, to the reaction mixture was added 20% aqueous hydrochloric acid solution (30 ml) and the solution was heated under reflux for 5 hours. The reaction mixture was concentrated to dryness under reduced pressure. The residue was dissolved in hot water (50 ml), treated with activated charcoal and filtered. The filtrate was diluted with ethanol (50 ml) and adjusted to pH 5 with aqueous ammonia and cooled. The resultant crystals were collected by filtration, washed with water and dried to afford 3-amino-3-(4-chloro-2-nitrophenyl)propionic acid (6.5 g, yield 76%) as pale yellow crystals M.p. 211—213°C (dec.).

(2) Preparation of 6-chloro-1H-indazol-3-ylacetic acid

Hydrazine (mono hydrate) (1.5 g) was added to a solution of 3-amino-3-(4-chloro-2-nitrophenyl)propionic acid (7.5 g) in 6% aqueous sodium hydroxide solution (42 ml). Raney nickel catalyst (about 10 mg) was added to the solution at 75°C with vigorous stirring and the mixture was stirred at 75—80°C for 10 minutes. After additional Raney nickel (about 10 mg) was added to the mixture, the stirring was continued at the same temperature until evolution of nitrogen gas ceased (it took about 20 minutes). The resultant mixture was treated in substantially the same manner as described in Example 1 to afford 6-chloro-1H-indazol-3-ylacetic acid (5.9 g, yield 91%), as colorless crystals M.p. 180—182°C (dec.).

## Example 3

*Preparation of 4-chloro-1H-indazol-3-ylacetic acid*

Hydrazine (mono hydrate) (0.5 g) was added to a solution of 3-amino-3-(2-chloro-6-nitrophenyl)propionic acid (4 g) in 5% aqueous sodium hydroxide solution (25 ml). Raney nickel catalyst (about 5 mg) was added to the solution at 80°C with vigorous stirring and the mixture was stirred at 80°C for 15 minutes. After additional catalyst (5 mg) was added to the mixture, the stirring was continued at the same temperature until evolution of nitrogen gas ceased (it took about 30 minutes). The resultant mixture was treated in substantially the same manner as described in Example 1 to give 4-chloro-1H-indazol-3-ylacetic acid (1 g, yield 30.0%) as colorless crystals M.p. 205—206°C (dec.).

## Example 4

*Preparation of 7-methoxy-1H-indazol-3-ylacetic acid*

(1) Preparation of 3-amino-3-(3-methoxy-2-nitrophenyl)propionic acid

3-Methoxy-2-nitrobenzaldehyde (9.3 g), malonic acid (6.9 g) and ammonium acetate (9.8 g) were dissolved in a mixture of formic acid (10 ml) and acetic acid (20 ml). The solution was stirred at 55—60°C for 2 hours, at 90—95°C for 3 hours and then heated under reflux for an hour. Then, to the reaction mixture was added 20% aqueous hydrochloric acid solution (30 ml) and the resulting solution was heated under reflux for 2 hours. The resultant solution was concentrated to dryness under reduced

pressure and the residue was dissolved in hot water (100 ml), treated with activated charcoal and filtered. The filtrate was diluted with ethanol (100 ml) and adjusted to pH 5 with aqueous ammonia and cooled to give crystals. The crystals were collected by filtration, washed with water and dried to give 3-amino-3-(3-methoxy-2-nitrophenyl)propionic acid (8.9 g, yield 72%) as pale yellow crystals M.p. 218°C (dec.).

(2) Preparation of 7-methoxy-1H-indazol-3-ylacetic acid

Hydrazine (mono hydrate) (0.5 g) was added to a solution of 3-amino-3-(3-methoxy-2-nitrophenyl)propionic acid (2 g) in 4.5% aqueous sodium hydroxide solution (21 ml). Raney nickel catalyst (about 5 mg) was added to the solution at 75—80°C with vigorous stirring and the mixture was stirred at the same temperature for 15 minutes. The resultant mixture was treated substantially in the same manner as described in Example 1 to give 7-methoxy-1H-indazol-3-ylacetic acid (0.9 g, yield 52.4%) colorless crystals M.p. 180—181°C (dec.).

Example 5

*Preparation of ethyl 5-chloro-1H-indazol-3-ylacetate*

(1) Preparation of 3-amino-3-(5-chloro-2-nitrophenyl)propionic acid

Formic acid (100 g), 5-chloro-2-nitrobenzaldehyde (100 mg) and malonic acid (73.2 g) were mixed and heated at 40—45°C. To the solution was added ammonium formate (85.2 g) at the same temperature. The solution was stirred at 60—70°C for 1 hour and at 90—95°C for 4 hours, and then heated at 115°C under reflux for 1 hour. To the solution were added 35% aqueous hydrochloric acid solution (192 g) and water (83 ml), and the solution was heated at 110°C under reflux for 1 hour. The resultant solution was cooled to 90—95°C, and then to the solution were added water (320 ml) and methyl isobutyl ketone (320 g). The mixture was stirred at 50—60°C and the aqueous layer was separated. The methyl isobutyl ketone layer was washed with water (100 g) and the aqueous layer was combined to the above one. The combined aqueous layers were adjusted to pH 5—6 with 48% aqueous sodium hydroxide solution below 50°C, cooled and left to stand over night below 10°C. The obtained crystals were collected by filtration and washed with water (300 ml) to give 3-amino-3-(5-chloro-2-nitrophenyl)propionic acid (110 g, yield 77%). M.p. 209—211°C (dec.).

(2) Preparation of 5-chloro-indazol-3-ylacetic acid

3-Amino-3-(5-chloro-2-nitrophenyl)propionic acid (42.8 g) and sodium hydroxide (21 g) were dissolved in water (195 ml). To the solution was added activated charcoal (4.7 g) and hydrazine (mono hydride) (18.5 g) at 30—40°C and the mixture was stirred at the same temperature for 15 minutes. Then, the solution was heated to 60—65°C and stirred at the same temperature for 1 hour and 80—85°C for 3 hours. To the reaction mixture was added water (156 ml) and the resulting mixture was added dropwise to 35% aqueous hydrochloric acid solution (81 g) over the course of 30 minutes. The obtained crystals were collected by filtration, washed with water (200 ml) and dried to give 5-chloro-indazol-3-ylacetic acid (37.0 g).

(3) Preparation of ethyl 5-chloro-1H-indazol-3-ylacetate

The above obtained 5-chloro-1H-indazol-3-ylacetic acid (37 g) was added to a mixture of ethanol (222 ml) and sulfuric acid (1.8 g). The mixture was heated under reflux for 6 hours and then cooled to below 10°C. To the reaction mixture was added 24% aqueous sodium hydroxide solution (5.4 ml) and the resulting mixture was adjusted to pH 4—5. The activated charcoal was filtered and washed with ethanol. The filtrate and the washings were combined and concentrated under reduced pressure. The concentrate was cooled to 25—30°C, to which sodium bicarbonate (3 g) was added. After cooled below 5°C, to the mixture was added water (230 ml) little by little over the course of 2 hours. The obtained crystals were collected by filtration, washed with ice water (80 ml) and then dried to give ethyl 5-chloro-1H-indazol-3-ylacetic acetate as crystals (33.4 g, total yield from 5-chloro-2-nitrobenzaldehyde 68%). M.p. 76—77°C.

**Claims**

1. A process for preparing an 1H-indazol-3-ylacetic acid derivative of the formula:

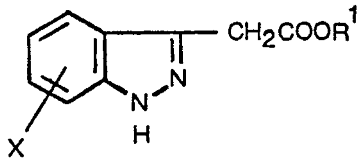

wherein $R^1$ is a hydrogen atom or a $C_1$ to $C_6$ alkyl group, and X is a hydrogen atom, a halogen atom or a $C_1$ to $C_6$ alkoxy group, or its salt which is characterized by reacting an aminoacetic acid derivative of the formula:

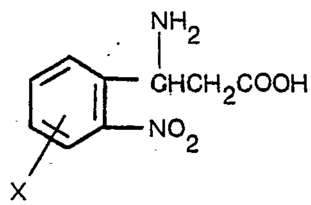

wherein X is as defined above, or its salt with a hydrazine compound to give a compound of the formula:

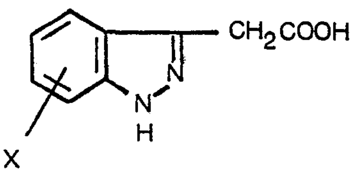

wherein X is as defined above or its salt, and if desired esterifying the compound to give a compound of the formula:

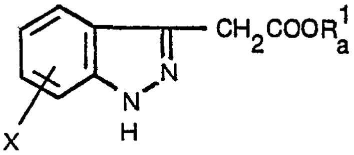

wherein $R^1_a$ is a $C_1$ to $C_6$ alkyl group and X is as defined above.

2. A process of claim 1, wherein the reaction of the amino-acetic derivative with a hydrazine compound is conducted in the presence of catalyst selected from activated charcoal, Raney nickel, palladium charcoal, platinum oxide and colloidal platinum.

3. A process of claim 2, wherein the catalyst is selected from Raney nickel and activated charcoal.

4. A process of claim 1, wherein $R^1$ is ethyl and X is 5-chlorine.

5. A process of claim 2, wherein $R^1$ is ethyl and X is 5-chlorine.

6. A process of claim 3, wherein $R^1$ is ethyl and X is 5-chlorine.

## Revendications

1. Procédé de préparation d'un dérivé d'acide 1H-indazol-3-ylacétique ayant la formule:

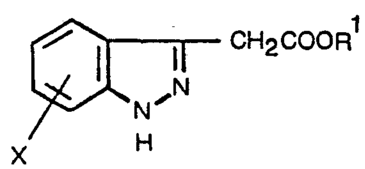

où $R^1$ est un atome d'hydrogène, ou un groupe alkyle en $C_1$ à $C_6$, et X est un atome d'hydrogène, un atome d'halogène ou un groupe alcoxy en $C_1$ à $C_6$ ou son sel, caractérisé en ce qu'on fait réagir un dérivé d'acide aminoacétique ayant la formule:

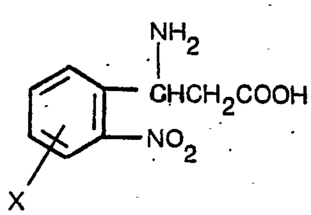

où X est tel que défini ci-dessus ou son sel, avec un composé d'hydrazine pour donner un composé ayant la formule:

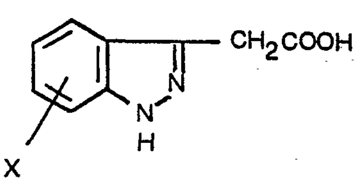

où X est tel que défini ci-dessus ou son sel, et si on le désire, on estérifie le composé pour donner un composé ayant la formule:

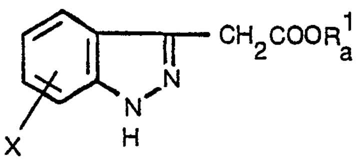

où $R_a^1$ est un groupe alkyle en $C_1$ à $C_6$ et X est tel que défini ci-dessus.

2. Procédé selon la revendication 1, caractérisé en ce que la réaction du dérivé aminoacétique avec un composé d'hydrazine est conduite en présence d'un catalyseur choisi parmi le charbon activé, le nickel de Raney, le palladium-charbon, l'oxyde de platine et le platine colloïdal.

3. Procédé selon la revendication 2, caractérisé en ce que le catalyseur est choisi parmi le nickel de Raney et le charbon activé.

4. Procédé selon la revendication 1, caractérisé en ce que $R^1$ est le groupe éthyle et X est le chlore en position 5.

5. Procédé selon la revendication 2, caractérisé en ce que $R^1$ est le groupe éthyle et X est le chlore en position 5.

6. Procédé selon la revendication 3, caractérisé en ce que $R^1$ est le groupe éthyle et X est le chlore en position 5.

**Patentansprüche**

1. Verfahren zur Herstellung eines 1H-Indazol-3-ylessigsäure-Derivats der Formel

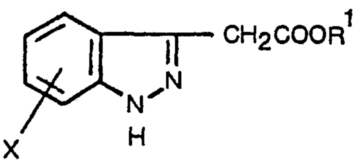

worin $R^1$ ein Wasserstoffatom oder eine $C_1$—$C_6$-Alkylgruppe und X ein Wasserstoffatom, ein Halogenatom oder eine $C_1$—$C_6$-Alkoxygruppe bedeuten, oder seines Salzes, das dadurch gekennzeichnet ist, daß man umsetzt ein Aminoessigsäurederivat der Formel

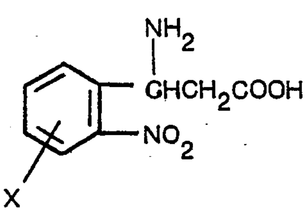

worin X wie oben definiert ist, oder sein Salz mit einer Hydrazinverbindung unter Bildung einer Verbindung der Formel

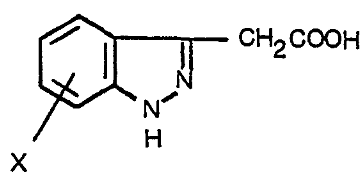

worin X wie oben definiert ist, oder ihres Salzes, und gewünschtenfalls die Verbindung verestert unter Bildung einer Verbindung der Formel

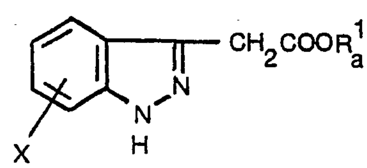

worin $R_a^1$ eine $C_1$—$C_6$-Alkylgruppe bedeutet und X wie oben definiert ist.

2. Verfahren nach Anspruch 1, worin die Umsetzung des Aminoessigsäure-Derivats mit einer

Hydrazinverbindung in Gegenwart eines Katalysators durchgeführt wird, der ausgewählt wird aus Aktivkohle, Raney-Nickel, Palladium-Kohle, Platinoxid und kolloidalem Platin.

3. Verfahren nach Anspruch 2, worin der Katalysator ausgewählt wird aus Raney-Nickel und Aktivkohle.

4. Verfahren nach Anspruch 1, worin $R^1$ Äthyl und X 5-Chlor bedeuten.

5. Verfahren nach Anspruch 2, worin $R^1$ Äthyl und X 5-Chlor bedeuten.

6. Verfahren nach Anspruch 3, worin $R^1$ Äthyl und X 5-Chlor bedeuten.